# EUROPEAN PATENT APPLICATION

(11) **EP 1 245 250 A2**
(43) Date of publication of application: **02.10.2002**
(21) Application number: 02252114.0
(22) Date of filing: 25.03.2002
(51) Int. Cl.: A62B 9/00

(54) **Oxygen sensor mounting in medical or flight crew masks for direct indication of blood oxygen level**

(30) Priority: 29.03.2001 US 821229
(71) Applicant: ROSEMOUNT AEROSPACE INC., Burnsville, MN 55306-4898 (US)
(72) Inventor: Bachinski, Thomas J., Lakeville, Minnesota 55044 (US)
(74) Representative: Cross, Rupert Edward Blount

(57) **Abstract**

The present invention relates to sensing conditions of blood of the wearer (10) of an oxygen mask (12) that provides supplemental oxygen to the wearer (10). A sensor (20) is mounted on the interior of the mask (12) directly adjacent to or in contact with the skin (22) of the wearer (10). The sensor (20) preferably is positioned just below the jaw in the throat area, where blood vessels are relatively close to the skin (22) and are of sufficient number and size so that a condition such as blood oxygen level can be sensed by non-invasive sensors (20). The sensor (20) in turn is connected to a suitable controller (24) that will receive the signal from the sensor (20) and control the flow of oxygen to the mask (12), both as to pressure, and to control oxygen level. Additionally, the controller (24) can regulate outside conditions such as a pressure suit (42) that may be worn by a fighter pilot, or can activate alarms (44) as desired.

## Description

### BACKGROUND OF THE INVENTION

When pilots are operating high performance aircraft, as well as for medical purposes when a patient is receiving supplemental oxygen, it is desirable to monitor the oxygen level in the blood. The present invention relates to monitoring oxygen levels whenever an oxygen mask is worn.

Presently non-invasive sensors that utilize light beams directed at the skin in regions where there are blood vessels adjacent to the skin are available. The sensors are available for use on various parts of the body, such as finger pulse meters, and will determine the oxygen level in the blood without actually drawing a sample of the blood. Such devices are made by Nonin Medical, Inc. of Plymouth, Minnesota.

The non-invasive sensors are maintained in a small housing, with the components self contained, as in the present invention. The readings can be obtained remotely. Suitable software is used in the control of these units.

The users of oxygen masks have a special need for monitoring since supplemental oxygen is being used.

### SUMMARY OF THE INVENTION

The present invention provides an oxygen mask for use on a human including a chin portion along a lower level of the oxygen mask, a sensor for monitoring blood conditions mounted on an interior surface of the chin portion, the sensor sensing conditions of blood in vessels in the skin of a person wearing the mask.

The present invention also provides a mask for covering the nose and mouth of a user and for supplying a gas for breathing by the user, said mask having a breath cavity and being shaped so that portions of the mask contact a user for support, the mask having a gas supply tube leading to the breath cavity, and a sensor mounted on an interior surface of the mask in a position to sense blood in vessels under the skin of a user, a controller coupled to the sensor for receiving signals from the sensor that indicate a condition of the blood being sensed, and a gas regulator connected to the controller, the controller controlling the gas regulator to regulate the gas provided through the gas supply tube in response to signals from the sensor.

The present invention relates to the mounting of a blood oxygen level sensor that will non-invasively determine the oxygen content in blood that is flowing in vessels beneath the skin directly in an oxygen mask. Specifically, the sensor is mounted in a location that insures substantially instantaneously monitoring blood oxygen level whenever a person is using such oxygen mask. The blood vessels beneath the jaw of a person, in the throat area, are close to the skin surface and accessible for non-invasive analyzation of the blood oxygen. The oxygen masks, particularly for pilots must be close fitting and comfortable, and the location below the jaw can accommodate such sensors without discomfort. The sensor in the mask is positioned so that whenever the mask is worn the sensor is in contact with or closely adjacent to the skin below the jaw and insures that any changes in blood oxygen, which can indicate conditions that are in need of correction, will be indicated. For example, in aircrew or pilot monitoring, G-forces or conditions which would indicate that the oxygen provided to the brain is low, which can cause the person to pass out, can be sensed. The output signal can be used for controlling and actuating systems to correct the matter, such as increasing the pressurization of a pressurized suit being used, or increasing the pressure and/or flow of the oxygen that is being provided to the person. In general the signals indicating low or lowering blood oxygen levels, or increasing levels that also can cause problems, can be used for initiating corrective action.

The level of oxygen in the blood can also indicate hypoxia, stress, and other conditions that are brought about by low oxygen, as well as high oxygen content.

The concept of locating the sensor in the face mask that is worn by persons requiring oxygen insures that the blood oxygen level parameter will be monitored whenever there is a need for supplemental oxygen.

Other non-invasive blood parameters can be sensed by suitable sensors located in the oxygen mask, such as pulse rate, blood pressure, and similar functions which are affected by the patient's condition.

### BRIEF DESCRIPTION OF THE DRAWINGS

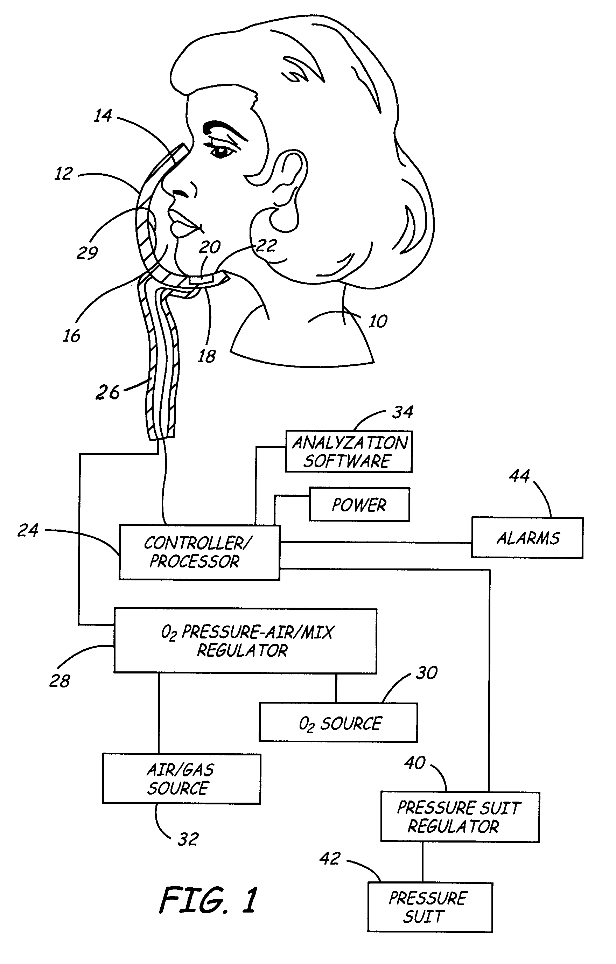

The single Figure is a schematic cross sectional view of an oxygen mask in place on a user, and a block diagram of the controls utilized.

### DETAILED DESCRIPTION OF THE PREFERRED EMBODIMENTS

Referring to the Figure a person indicated generally at 10, such as a fighter pilot, or a medical patient, is fitted with a conventional oxygen mask 12, which is shown only schematically, but it is shown in cross-section generally in the position worn. The actual positions of the oxygen masks may vary, and the outer configurations of such masks also varies. The mask includes a nose portion 14, a breathing cavity 16, and an under chin or under jaw wall 18. The under chin wall is relatively flexible, and as shown, a blood oxygen sensor 20 is mounted on the interior of the chin wall 18, and rests against or closely adjacent to the throat skin or throat area 22, directly under the jaw, as shown, of the person 10. The sensor is a conventional non-invasive blood oxygen sensor such as those made by Nonin Medical, Inc. of Plymouth, Minnesota. The type of sensor can be a pulse oximeter that shines red and infrared light through tissue and detects the fluctuating signals caused by arterial blood pulses. The ratio of the fluctuation of the red and infrared light signals received determines the oxygen saturation content.

The sensor is activated with a power source provided from a controller/processor 24. The blood oxygen sensor 20 provides signals that are processed in the controller/processor 24, that is connected to the sensor through the inlet tube or umbilical 26 of the mask. The inlet tube 26 is connected to a regulator 28 that is in turn connected to an oxygen source 30 and an air or other gas (nitrogen) source 32. The regulator 28 not only will control the pressure that is applied through the breathe tube 26 to the interior breathe cavity 16 of the mask, but also will properly mix the oxygen with nitrogen or other gas, or air for obtaining the appropriate ratio that is necessary or desirable for the person 10 that is receiving the oxygen.

The controller/processor 24 receives the signals from the sensor 20, and analyzes it with analyzation software 34, which is presently used in connection with handheld non-invasive oxygen sensors sold by Nonin Medical, Inc.

If the blood oxygen level of the person 10 drops, as sensed by the sensor 20 analyzing blood vessels in the throat or under chin area 22, the output from the processor can adjust the pressure regulator 28, and the air/mix regulator so that an appropriate enrichment of oxygen can occur, or the oxygen pressure being provided can be increased.

It also should be noted that the sensor 20 may be combined with a presently available carbon dioxide detector 29, that is also capable of determining the carbon dioxide level in blood by analyzing the breath in cavity 16. The carbon dioxide sensor 29 is non-invasive. the Sensor 29 is energized from the controller 24 and provides a signal usable for insuring that the person 10, whether a pilot or a patient, receives appropriate oxygen.

In the case of a pilot, the high G-forces can affect the flow of oxygen to the brain, and this can be reflected by information determined by the sensor 20. If the oxygen level drops, the processor 24 can provide a signal to a pressure regulator 40 that controls pressure to a pressure suit 42 that the person 10 is wearing. In addition, alarms 44 can be sounded or those signals can be used for activating equipment that will place the aircraft into a less stressful attitude, or the like.

In addition to under the chin positions, the sensor can be placed adjacent the jaw, or in other places where there are blood vessels of sufficient size, closeness to the skin surface, and of sufficient blood flow for the sensor to work correctly.

The type of oxygen mask is only shown schematically. The present invention will work by replacing the oxygen level sensor or other sensor that does non-invasive testing of blood vessels, in a location such as below the chin, which is preferred, or along the jaw where the oxygen mask would be in contact with the skin, or in the side along the cheek of the wearer.
The portion of the sensor that is in the mask can be made very thin, and can be covered with a suitable non-irritating cover material to insure that there is adequate comfort for the user.

Although the present invention has been described with reference to preferred embodiments, workers skilled in the art will recognize that changes may be made in form and detail without departing from the spirit and scope of the invention.

## Claims

1. An oxygen mask for use on a human including a chin portion along a lower level of the oxygen mask, a sensor for monitoring blood conditions mounted on an interior surface of the chin portion, the sensor sensing conditions of blood in vessels in the skin of a person wearing the mask.

2. The oxygen mask of claim 1, wherein the oxygen mask has an oxygen supply tube connected thereto, a controller remote from the sensor connected to the sensor by lines passing through the tube, said controller providing an output indicating the status of conditions being sensed by the sensor.

3. The oxygen mask of claim 2, wherein said inlet tube is connected to a source of a gas including oxygen, and the controller is connected to the source of gas to control a parameter of supply to the mask.

4. The oxygen mask of either of claims 2 or 3, wherein the sensor senses blood oxygen, and wherein oxygen is supplied to the oxygen mask from a pressure regulator, the controller being responsive to the output of the sensor for controlling the pressure of the oxygen being provided to the mask.

5. The oxygen mask of claim 4, further comprising a pressure suit regulator for regulating the pressure in a suit worn by the person wearing the mask, said pressure suit regulator being connected to the controller to regulate the pressure of the pressure suit responsive to the condition sensed by the sensor.

6. The oxygen mask of claim 1, wherein said sensor senses oxygen level in the blood, and a controller receiving signals from the sensor for controlling the flow of gas to the mask.

7. A mask for covering the nose and mouth of a user and for supplying a gas for breathing by the user, said mask having a breath cavity and being shaped so that portions of the mask contact a user for support, the mask having a gas supply tube leading to the breath cavity, and a sensor mounted on an interior surface of the mask in a position to sense blood in vessels under the skin of a user, a controller coupled to the sensor for receiving signals from the sensor that indicate a condition of the blood being sensed, and a gas regulator connected to the controller, the controller controlling the gas regulator to regulate the gas provided through the gas supply tube in response to signals from the sensor.

8. The mask of claim 7, wherein said gas for breathing comprises oxygen, and the controller controls the oxygen pressure supplied to the mask.

9. The mask of claim 7 or claim 8, wherein said regulator further regulates a mixture of oxygen with other gases provided to the mask.

10. The mask of any one of claims 7 to 9, wherein said controller further comprises connections to a pressure suit regulator for regulating pressure of a pressurized suit worn by a person wearing the mask.

11. The mask of any one of claims 7 to 10, further comprising a carbon dioxide sensor in the mask to sense gas in the breath cavity, the carbon dioxide sensor being coupled to the controller.
